# EUROPEAN PATENT SPECIFICATION

(11) **EP 2 879 587 B1**
(45) Date of publication and mention of the grant of the patent: **15.07.2020**
(21) Application number: 13750200.1
(22) Date of filing: 31.07.2013
(51) Int. Cl.: A61B 10/02, A61B 17/34

(54) **DEPTH CONTROLLED JAMSHIDI NEEDLE**
TIEFENGESTEUERTE JAMSHIDI-NADEL
AIGUILLE JAMSHIDI À PROFONDEUR CONTRÔLÉE

(30) Priority: 31.07.2012 US 201261677684 P
(43) Date of publication of application: 10.06.2015
(73) Proprietor: Orthovita, Inc., Malvern, PA 19355 (US)
(72) Inventor: GEIST, Wyatt, Drake, Davie, FL 33328 (US); NUNLEY, Pierce, Dalton, Shreveport, LA 71101 (US)
(74) Representative: Regimbeau
(86) International application number: PCT/US2013/053038
(87) International publication number: WO 2014/022567

(56) References cited:
- WO-A2-2010/138895
- GB-A- 2 403 419
- US-A1- 2005 131 345
- US-A1- 2007 260 255
- US-A1- 2010 185 161

## Description

### FIELD OF THE INVENTION

The present invention relates to instruments employed for controlling accurate placement of devices, such as Jamshidi needles, used in spinal surgery. In particular, in addition to enabling a surgeon to accurately control the depth of needle penetration into the bone, the present device properly places dilation tubes for percutaneous surgical procedures.

### BACKGROUND OF THE INVENTION

Medical procedures involving the vertebrae are normally complicated because of the preciseness and accuracy required to avoid both neural damage and injury to major blood vessels. Precision depth guided instruments are required to perform percutaneous spinal surgery. These surgeries sometimes require penetration of the hard cortical bone of the vertebra and traversal of the softer cancellous bone lying thereunder. A large force is normally required by the surgeon to penetrate the cortical bone. Once the cortical bone is penetrated, extreme care must then be taken to avoid rapidly penetrating through all of the cancellous bone. There is also the danger of rapidly passing through the cancellous bone and then through the cortical bone on the other side of the vertebra. This can result in injury or damage to the spinal cord and/or other organs or blood vessels located adjacent the spine. In some instances, the force required to penetrate the cortical bone is greater than a surgeon can apply by hand. In these instances a hammer or other similar instrument is required to force the instrument through the cortical bone. When a hammer or similar instrument is used, there is a greater danger of the instrument passing rapidly through the cancellous bone and out the other side of the vertebra. Preexisting conditions such as osteoporosis may complicate this problem.

### DESCRIPTION OF THE PRIOR ART

U.S. Patent No. 5,458,579 discloses an apparatus for inserting a trocar/cannula assembly through a wall of an anatomical cavity of an individual. The apparatus includes a housing for holding the trocar/cannula assembly, a device for driving the trocar/cannula assembly into the individual, a spine, and a depth stop element mounted on the spine to control the depth which the trocar/cannula assembly is inserted into the individual.

U.S. Patent No. 6,033,411 discloses a depth guided instrument for use in performing percutaneous implantation of hard tissue implant materials. A depth guided stylet includes a point adapted for piercing hard tissue and self-tapping threads for self tapping into hard tissue. The instrument also includes a cannula surrounding the stylet which employs a pawl and rack of gear teeth to assist passing the cannula through the hard tissue.

U.S. Patent No. 7,678,077 discloses an instrument for injecting therapeutic and other agents into an individual at a target site. The instrument includes a catheter having a first elongate shaft and a second elongate shaft slidingly disposed within the first shaft. An indicator is secured to an end of the second elongate shaft and moves relative to a scale to indicate the position of the first and second shafts relative to their insertion into an individual US 2007/260255 A1 discloses a bone penetrating assembly comprising: an outer cannula member, an inner cannula member and a needle member removably secured within said inner cannula member so that a tip portion of said needle member extends beyond a distal end of said inner cannula member.

None of the above noted prior art devices permit the operator of the device to adjust the depth of penetration of the Jamshidi needle or cannula based on information obtained from a patient prior to surgery. The depth of penetration can be adjusted depending on the point of entry into the body of the Jamshidi needle or cannula. In addition, none of these devices are constructed to place a tap tube or dilation tube simultaneously to placement of the needle.

### SUMMARY OF THE INVENTION

A precision depth guided instrument, such as a Jamshidi needle, is provided for use in various surgeries related to the vertebrae. The invention is defined by claim 1. Preferred embodiments of the invention are defined by the dependent claims.

The instrument includes an outer cannula, an inner cannula and a stylet. After the cortical bone of a vertebra is penetrated by the outer cannula of the instrument, the depth of penetration of the inner cannula is adjusted by rotation of a stop mounted to the outer cannula. The inner cannula is then moved further into the vertebrae, and a stop mounted on the outer cannula controls the depth of penetration of the inner cannula. The correct depth of penetration is determined by radiography prior to and during the procedure. In at least one embodiment, the outer cannula is provided with at least one tap or dilation tube which may be placed within the patient for use in subsequent surgical operations after withdrawal of the jamshidi needle.

The jamshidi needle may cooperate with the tap or dilation tubes to facilitate controlled withdrawal of the jamshidi from the cortical bone.

Accordingly, it is an objective of the instant invention to provide a depth controlled jamshidi needle which can be inserted into a patient to a precise location.

It is a further objective of the instant invention to provide a depth controlled jamshidi needle which can be inserted into a vertebra and then a portion of the device controllably moved to a precise location within the vertebra.

It is yet another objective of the instant invention to provide a depth controlled jamshidi needle which can be inserted into a vertebra a measured distance, the measured distance having been predetermined by radiography.

It is a still further objective of the invention to provide a depth controlled jamshidi needle which can be inserted into a vertebra an exact distance without any danger of exceeding the desired distance.

It is yet a further objective of the invention to provide a depth controlled jamshidi needle that includes at least one dilation tube positioned about the outer cannula which may be placed simultaneously with placement of the jamshidi needle.

It is still yet a further objective of the invention to provide a depth controlled jamshidi needle that includes at least one tap tube and at least one dilation tube positioned about the outer cannula which may be placed simultaneously with placement of the jamshidi needle.

Other objects and advantages of this invention will become apparent from the following description taken in conjunction with any accompanying drawings wherein are set forth, by way of illustration and example, certain embodiments of this invention. Any drawings contained herein constitute a part of this specification and include exemplary embodiments of the present invention and illustrate various objects and features thereof.

### BRIEF DESCRIPTION OF THE FIGURES

FIGURE 1 is a perspective view of one embodiment of the present invention with the components partially exploded;
FIGURE 2 is an exploded view of one embodiment of the present invention;
FIGURE 3 is a perspective view of one embodiment of the present invention illustrated with a component placement prior to insertion into a patient;
FIGURE 4 is a perspective view of one embodiment of the present invention illustrated with an inner cannula moved to position for penetration further into a vertebra;
FIGURE 5 is a partial view of the end portion of the embodiment illustrated in FIGURE 3 illustrating the relationship of the two cannulae and needle;
FIGURE 6 is a top view of a handle of the inner cannula into which a needle is inserted, illustrated with a perspective view of the outer cannula;
FIGURE 7 is a cross sectional view of one embodiment of the present invention illustrated positioned in a vertebra;
FIGURE 8 is a perspective view of one embodiment of the present invention illustrated in a position similar to FIGURE 3;
FIGURE 9 is a perspective view of one embodiment of the present invention illustrated in a position similar to FIGURE 4;
FIGURE 10 is a perspective view of one embodiment of the present invention illustrated with an optional hard stop;
FIGURE 11 is a perspective view of the invention illustrated in FIGURE 1 including an optional hard stop;
FIGURE 12 is a perspective view of one embodiment of the present invention illustrated with a tap tube and a dilation tube positioned about the outer cannula;
FIGURE 13 is a perspective partially exploded view of one embodiment of the present invention illustrating the tap tube partially in section;
FIGURE 14 is a perspective partially exploded view of the embodiment of FIGURE 12 illustrating the tap tube and dilation tube partially in section;
FIGURE 15 is a partial section view taken along lines 15-15 of FIGURE 12 illustrating connection between the tap and dilation tubes and the outer cannula;
FIGURE 16 is a perspective view of one embodiment of the present invention illustrated with the tap tube positioned about the outer cannula;
FIGURE 17 is a perspective view of the embodiment shown in FIGURE 16 illustrating partial removal of the outer cannula from the tap tube;
FIGURE 18 is a perspective view of the embodiment illustrated in FIGURE 16 illustrated with the tap tube removed from the outer cannula;
FIGURE 19 is an exploded perspective view of the embodiment illustrated in FIGURE 16;
FIGURE 20 is a perspective view of one embodiment of the present invention; and
FIGURE 21 is a partially exploded view of the embodiment illustrated in FIGURE 19.

### DETAILED DESCRIPTION OF THE INVENTION

While the present invention is susceptible of embodiment in various forms, there is shown in the drawings and will hereinafter be described a presently preferred, albeit not limiting, embodiment with the understanding that the present disclosure is to be considered an exemplification of the present invention and is not intended to limit the invention to the specific embodiments illustrated.

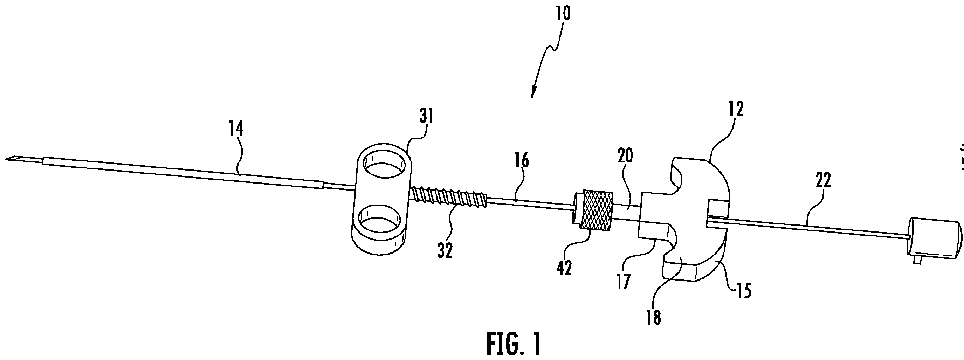

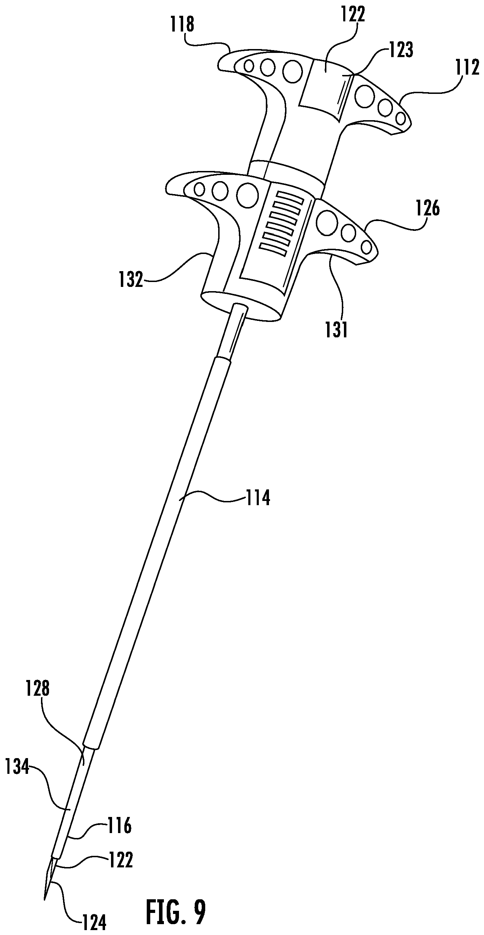

Figures 1-11, which are now referenced, illustrate various embodiments of the present invention and the manner in which they are assembled. Like reference numerals refer to like components in the various figures. The needle depth controlled Jamshidi assembly 10 comprises a Jamshidi-type needle 12, and an outer cannula assembly 14. The Jamshidi-type needle 12 is slidably disposed within the outer cannula assembly 14. The Jamshidi-type needle 12 includes an inner cannula 16 which is secured to a handle 18. The handle 18 includes a collar or cylindrical portion 20. This cylindrical portion 20 is fixedly secured to the cannula 16. The handle 18 preferably has an ergonomic shape that can comfortably fit into a surgeon's or medical technician's hand. The handle includes an upper curved portion 15 which is shaped to conform to an individual's palm. The lower portion 17 of the handle 18 is also curved. The curve of the lower portion 17 of the handle is designed to be grasped by the fingers of an individual to assist in the control of the Jamshidi-type needle 12. The handle 18 is used to drive the cannula into, and sometimes through bones of a vertebra. Sometimes the Jamshidi-type needle 12 can be driven through the bone only by using pressure exerted by an individual's hand. Other times a hammer or other instrument must be employed to drive the needle 12 through a bone. There is a risk that, when a hammer or similar instrument is utilized, the Jamshidi-type needle 12 will pass too far into a vertebra. This can cause damage to nerves located nearby. Sometimes the needle passes completely through the vertebra and injures an adjacent blood vessel or internal organ. To prevent this, the present invention utilizes a second outer cannula 14 which is adjustably secured to the Jamshidi-type needle 12 to provide controlled linear traversal of the Jamshidi-type needle within the outer cannula assembly 14.

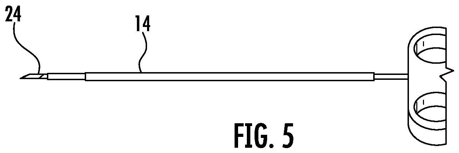

A needle 22 is slidably positioned within the inner cannula 16 of the Jamshidi-type needle 12. The preferred embodiment of the present invention illustrates the needle 22 as having a conical tip 24 (Figs. 5). However, other tips and needles can also be employed. For example, a trocar tip can be utilized. The tip 24 can be tapered, hollow, cannulated, etc. The tip can be utilized to extract a tissue sample. It can also be utilized to penetrate or anchor to a bone. In a preferred embodiment, the needle is detachable from the inner cannula so that a guidewire, Kirschner wire, K-wire or the like can be passed through the inner cannula to the surgical site. Alternatively, the Jamshidi needle can be removed and an orthopedic bone screw or other device can then be passed down the needle 22 and secured to the correct location on a bone. While the preferred embodiment of the present invention discloses a relatively rigid needle 22, other needles which are flexible can also be employed.

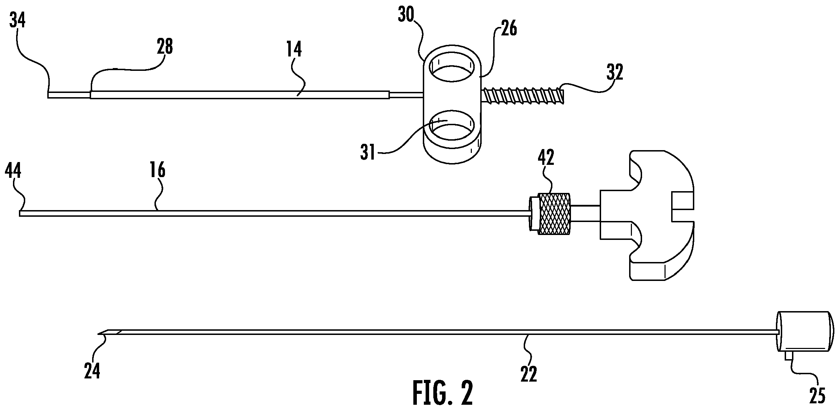

The outer cannula 14 comprises an upper portion 26 secured to a cannula 28 (Fig. 2). The upper portion 26 comprises a handle or grip 30 and a threaded sleeve 32. In the preferred embodiment of the outer cannula, the handle 30 and the threaded sleeve 32 are fixedly secured to each other. In other embodiments, these elements can be pivotably or removably secured to each other. The lower or second end 34 of the outer cannula 14 is constructed and arranged to penetrate and pass through bone. While the lower end 34 is normally a hollow tube with an end that tapers to a sharp edge, other edges can also be employed. For example, the edge can be serrated, saw toothed or sinusoidal. The smooth edge is preferably utilized when the needle assembly is driven straight into or through a bone. The serrations or waves are employed when additional effort is required to penetrate a bone. The handle or grip 30 is preferably provided with apertures 31 into which a surgeon's fingers can be inserted to control the device.

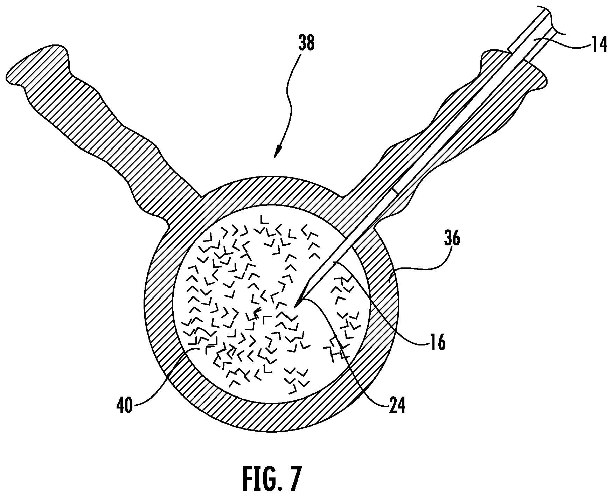

Figure 7 illustrates an example of one of the uses of the present invention. The outer cannula 14, the inner cannula 16 and the tip 24 of the needle 22 are passed through a cortical bone 36 of a vertebra 38 and into the cancellous bone 40. A sample of the cancellous bone may now be taken. In another situation, the needle 22 may be passed into the cortical bone 36 opposite the point of insertion into the vertebra. In these different situations it is very difficult for a surgeon or medical technician to judge the depth of penetration of the depth controlled Jamshidi type needle 10 into the vertebra. The remedy for this problem lies in the present invention. First, a radiography image of the vertebra being operated upon is taken. Next, the depth into the vertebra that the surgeon wants the needle to penetrate is measured or estimated. The outer cannula includes a first stop member 28. The outer cannula is driven into the bone until the first stop member 28 contacts the top surface of the bone, providing a reference depth that the outer cannula has penetrated the bone. The Jamshidi-type needle 12 of the present invention includes a threaded nut 42 or similar device which is rotatably secured to the cannula 16 by a bearing. The threads on the nut 42 match the threads on the sleeve 32. After the surgeon inserts the needle into a patient and through the cortical bone 36 of a vertebra, the cannula 16 is inserted further into the vertebra by rotating the nut 42. Rotation of the nut 42 moves the cannula and needle 22 further into the vertebra. The distance that these elements are moved can be measured along the threaded sleeve. Alternatively, since the threads have a fixed pitch, the surgeon need only count the rotations of the nut or inner cannula to determine the traversal of the inner cannula through the outer cannula and into the bone. The distance the needle needs to travel into the vertebra, which has been determined by radiography, can be accurately determined. Preferably, marks or indicia are placed on sleeve 32 which allow the surgeon to visually determine the position of the inner cannula with respect to the outer cannula. The surgeon or medical technician can now rotate the nut 42 until it reaches the desired mark of indicia on sleeve 32. At this point, the cannula 16 and needle 22 are now exactly where they need to be positioned. The nut can be rotated by hand or with a wrench or similar device. The wrench may be utilized if a relatively hard bone is to be penetrated by the device. Because the first stop member 28 is placed a predetermined distance from the distal tip of the outer cannula, the surgeon has a precise knowledge of how far the bone has been penetrated.

In an alternate embodiment, the nut 42 is fixedly secured to the collar 20 and handle or grip 30. In this embodiment the handle 30 is rotated, which in turn rotates the collar 20 and nut 42. This moves the cannula 16 further into the vertebra until the desired position is reached. Rotation of the handle 30 permits additional torque to be applied to the nut 42, and in certain instances does not required the use of a wrench or similar tool.

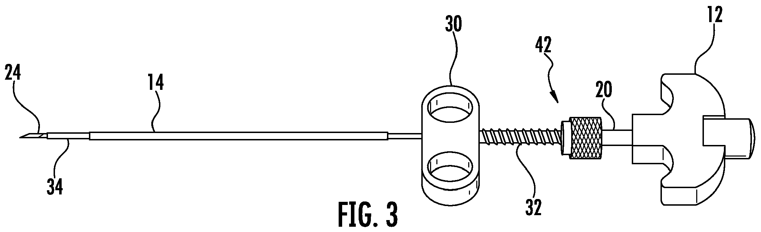

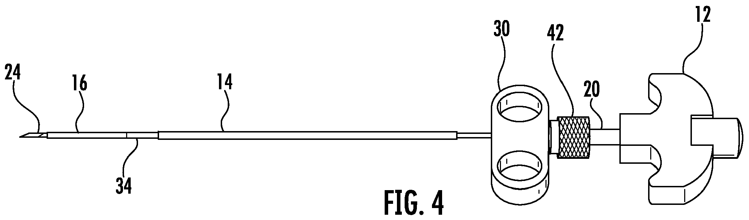

Figure 3 illustrates the position of the elements of the invention prior to rotation of nut 42. The end of cannula 16 is adjacent the end 34 of the outer cannula. The tip 24 of the needle 22 protrudes slightly past this point. As illustrated in Fig. 4, after nut 42 is rotated, the inner cannula 16 and needle 22 move past the end 34 of cannula 14. The distance that the inner cannula 16 and needle need to move past the end 34 of cannula 14 has been previously determined by radiography. The end 44 (Fig. 2) of inner cannula 16 can be similar in construction to end 34 of outer cannula 14. While the end 44 is normally a hollow tube with an end that tapers to a sharp edge, other edges can also be employed. For example, the edge can be serrated, saw toothed or sinusoidal. The smooth edge is preferably utilized when the needle assembly is driven straight into or through a bone. The serrations or waves are employed when additional effort is required to penetrate a bone.

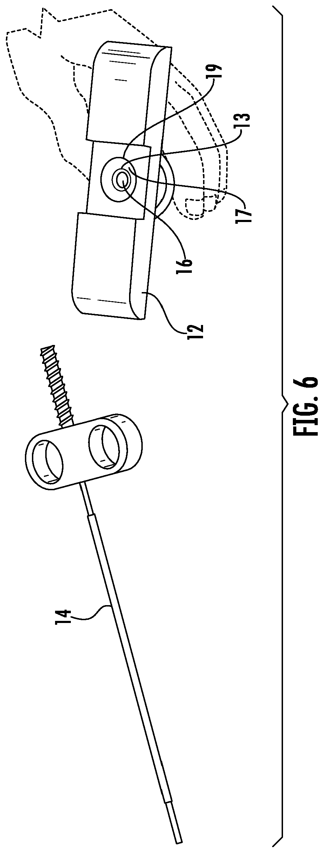

Figure 6 illustrates the hollow cannula 16 into which the needle 22 is slidably located. Also visible in Fig. 6 is funnel 17 which extends from the upper portion 15 of handle 18 to the first end 13 of the inner cannula 16. The funnel 17 includes an open mouth 19 which tapers substantially to the diameter of the inner bore of the inner cannula. The funnel provides the user with an easily targeted aperture for insertion of the needle 22 or a guidewire, also known as a Kirschner wire (not shown) often used in spinal as well as other types of surgeries.

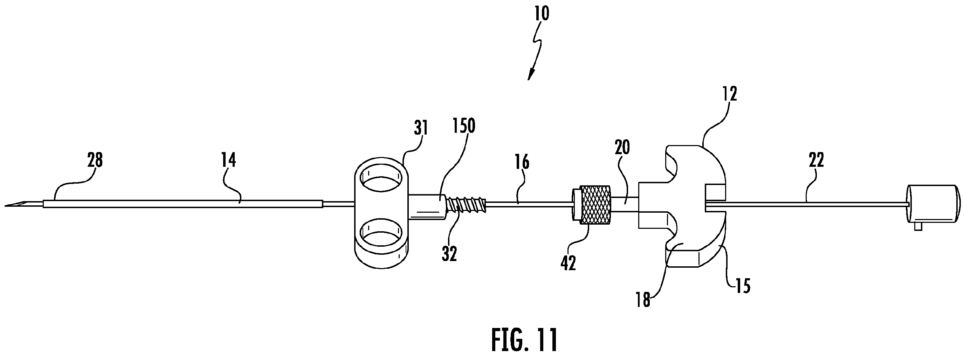

Figure 11 illustrates an optional collar or spacer 150 that can be placed on the sleeve 32. This spacer or collar 150 enables the surgeon or medical technician to precisely place the cannula 16 and needle 22 within a bone or other portion of a patient. The precise desired location of the cannula 16 and needle 22 is first determined by radiography. A measurement of this location is taken, and then a spacer or collar 150 is selected; the length of the spacer corresponding to this measurement. The spacer or collar is then placed over the sleeve 32 and the procedure is performed. The first stop member 28 determines the depth that the outer cannula 14 enters the bone and the inner cannula or nut 16, 42 is rotated to position the distal end of the needle 22 precisely where the surgeon or medical technician has determined it should be. This optional spacer prevents any over-insertion of the cannula 16 and needle 22.

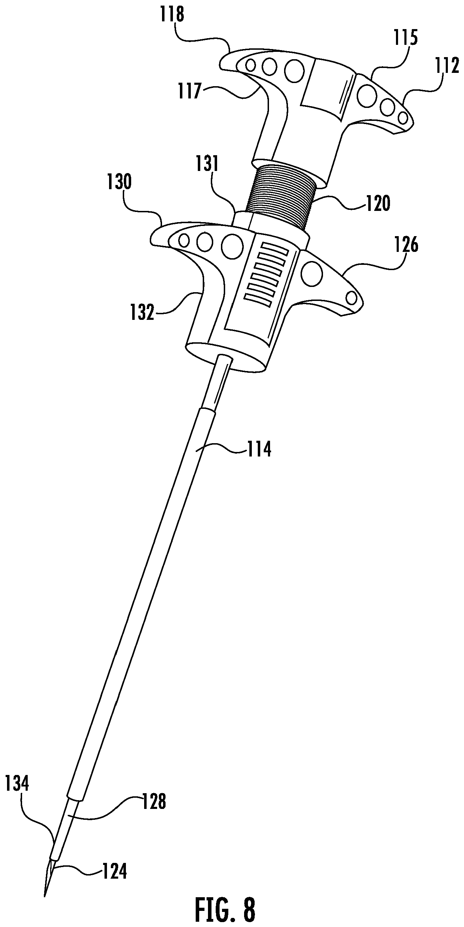

Figures 8-10 illustrate an alternate embodiment of the present invention. In these figures, a Jamshidi-type assembly 112 is slidably disposed within an outer cannula assembly 114. The Janshidi-type needle 112 includes a cannula 116 which is secured to a handle 118. The handle 118 includes a threaded collar portion 120. This threaded collar portion 120 is fixedly secured or molded to the handle 118. The handle 118 preferably has an ergonomic shape that can comfortably fit into a surgeon's or medical technician's hand. The handle includes an upper curved portion 115 which is shaped to conform to an individual's palm. The lower portion 117 of the handle 118 is also curved. The curve of the lower portion 117 of the handle is designed to be grasped by the fingers of an individual to assist in the control of the Jamshidi-type assembly 112. The handle 118 is used to drive the cannula into, and sometimes through bones of a vertebra. Sometimes the Jamshidi-type assembly 112 can be driven through the bone only by using pressure exerted by an individual's hand. Other times a hammer or other instrument must be employed to drive the Jamshidi-type assembly 112 through a bone. There is a risk, that when a hammer or similar instrument is utilized, the Jamshidi-type assembly 112 will pass too far into a vertebra. This can cause damage to nerves located nearby. Sometimes the needle passes completely through the vertebra and injures an adjacent blood vessel or internal organ. To prevent this, the present invention utilizes a second cannula 114 which is adjustably secured to the Jamshidi-type assembly 112.

A needle 122 is slidably positioned within the cannula 116. The needle, like the other embodiments includes an enlarged head member 123 which includes a boss 25 (Fig. 2) or the like suitable to secure the needle within the inner cannula assembly. In a most preferred embodiment, the boss cooperates with the handle 118 to function as a bayonet type mount. This embodiment of the present invention illustrates the needle as having a conical tip 124, however, other tips and needles can also be employed. For example, a trocar needle can be utilized. The tip 124 can be tapered, hollow, etc. The tip can be utilized to extract a tissue sample. It can also be utilized to anchor the needle to a bone. An orthopedic bone screw or other device can then be passed down the needle and secured to the correct location on a bone. While the preferred embodiment of the present invention discloses a relatively rigid needle, other needles which are flexible can also be employed. Alternatively, the bayonet mount may be utilized to remove the needle from the inner cannula for placement of a guidewire, K-wire or a Kirschner wire.

The outer cannula 114 comprises an upper portion 126 secured to a cannula 128 (Fig. 8). The upper portion 126 comprises a handle or grip 130. In this embodiment, the outer or second cannula 114 and the handle 130 are fixedly secured to each other. In other embodiments, these elements can be pivotably secured to each other. The lower end 134 of the outer cannula 114 is constructed and arranged to penetrate and pass through bone. While the end 134 is normally a hollow tube with an end that tapers to a sharp edge, other edges can also be employed. For example, the edge can be serrated, saw toothed or sinusoidal. The smooth edge is preferably utilized when the needle assembly is driven straight into or through a bone. The serrations or waves are employed when additional effort is required to penetrate a bone.

The Jamshidi-type assembly 112 of this embodiment includes a threaded collar portion 120. The second cannula 114 includes a housing 132 at a top end thereof. The housing is relatively hollow and includes threads which match the threads on sleeve portion 120. After the surgeon inserts the needle into a patient and through the cortical bone 36 of a vertebra, the cannula 116 is inserted further into the vertebra by rotating the handle 118 and sleeve portion 120. Rotation of sleeve 120 moves the cannula 116 and needle further into the vertebra. The distance that these elements are moved can be measured along the sleeve portion 120 which is provided with windows 162 and/or indicia 164. The distance the needle needs to travel into the vertebra, which has been determined by radiography, will be measured along sleeve portion 120. The surgeon or medical technician can now rotate handle 118 and sleeve 120 until it reaches the desired mark of indicia on sleeve 120. At this point, the cannula 116 and Jamshidi-type assembly 112 are now exactly where they need to be positioned.

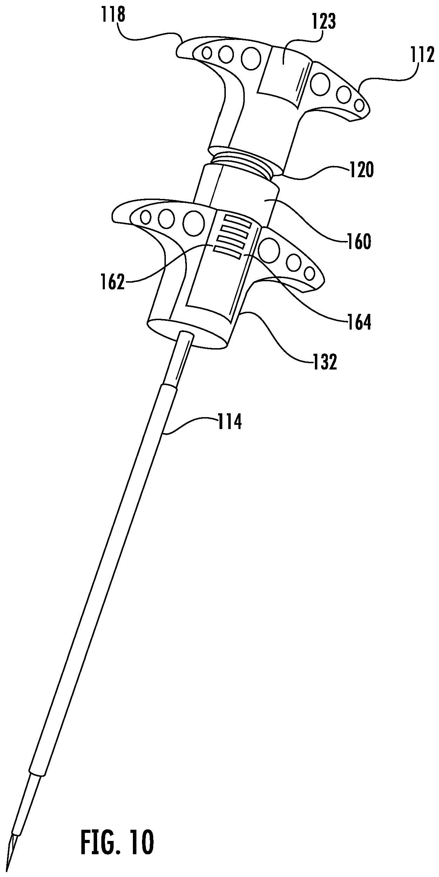

Fig. 10 illustrates an optional collar or spacer 160 that can be placed on the sleeve 120. This spacer or collar 160 enables the surgeon or medical technician to precisely place the cannula 116 and Jamshidi-type assembly 112 within a bone or other portion of a patient. The precise desired location of the cannula 116 and Jamshidi-type assembly 112 is first determined by radiography. A measurement is taken and then a spacer or collar 160 is selected which corresponds to this measurement. The spacer or collar is then placed over the sleeve 120 and the procedure is performed. This optional spacer prevents any over-insertion of the cannula 116 and Jamshidi-type assembly 112.

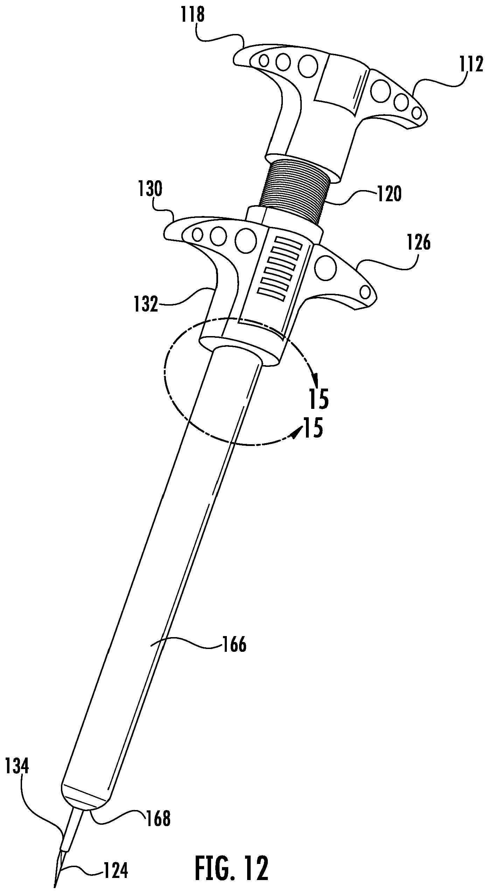

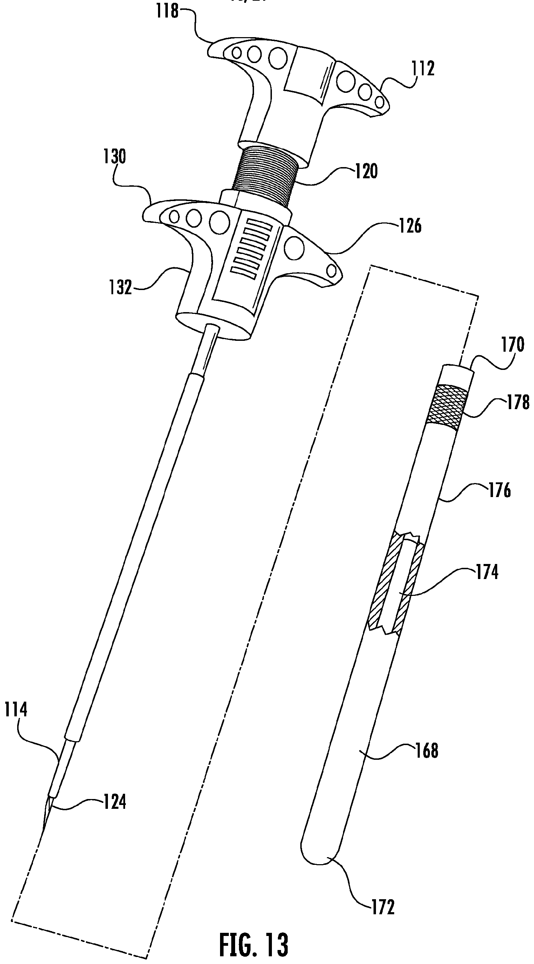

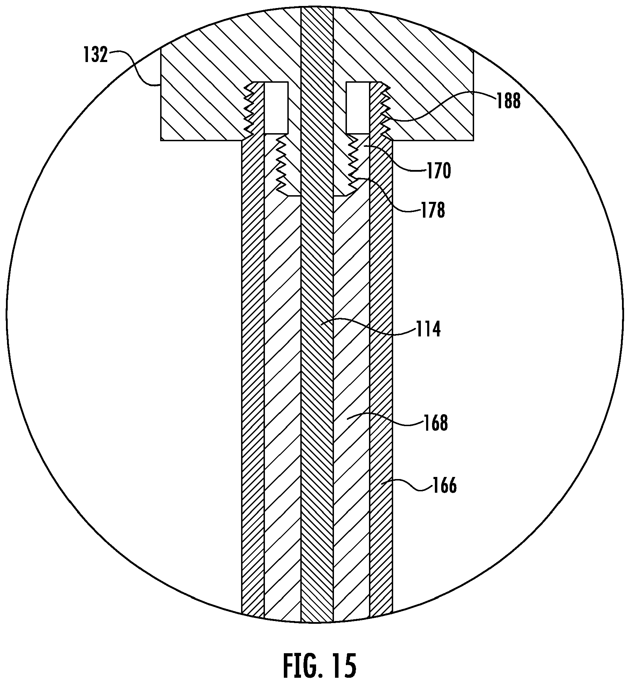

Now referring to Figs. 12-15, an alternative embodiment of the depth controlled jamshidi is illustrated. This embodiment includes at least one dilator tube 166 positioned about the outer cannula 114. In a most preferred embodiment, a pair of dilation tubes in the form of a tap tube 168 and a dilation tube 166 is positioned about the outer cannula 114. The tap tube includes a first end 170, a second end 172, an inner diameter 174 and an outer diameter 176. The tap tube generally includes a length that is slightly shorter than the outer cannula. In this manner, the tap tube can function as a stop member for the outer cannula, whereby the progression of the outer cannula through bone is stopped when the second end 172 of the tap tube contacts the outer surface of the bone. The inner diameter 174 of the tap tube 168 is sized to allow passage of a drill or tap member (not shown), thus the inner diameter also allows the tap tube to over the outer surface of the outer cannula. The first end 170 of the tap tube 168 preferably includes a connection to the outer cannula illustrated herein as internal threads 178 (Fig. 15). The second end 172 of the tap tube 168 is preferably tapered or radiused to allow for traversal through tissue *in vivo.* Thus, in operation, a surgeon can tap the depth controlled jamshidi needle 10 into a position wherein the second end 172 of the tap tube 168 contacts the bone. The surgeon can then rotate the handle 118 until the inner cannula 116 reaches the desired depth. The cap 123 of the needle 22 can be rotated to release the needle from the inner cannula 116. The needle is then withdrawn and a guidewire can be passed through funnel 17 and the inner cannula to the surgical site. The tap tube 168 can then be rotated to release it from the outer cannula 114. Gripping surface 178 provides the surgeon with sufficient friction to allow the tap tube to be released from the outer cannula. While the gripping surface 178 is illustrated as a knurled surface, other grippable surfaces may be utilized without departing from the scope of the invention; such gripping surfaces may include, but should not be limited to, wrench flats, an enlarged diameter, polygons and the like. The dilation tube 166 may be utilized in some embodiments over the outer diameter of the tap tube 168. The dilation tube includes a first end 180, a second end 182, an inner diameter 184 and an outer diameter 186. The first end 180 includes an attachment, illustrated herein as external threads 188 (Fig. 15) for attachment to the outer cannula. This construction allows the dilation tube 166 to be released as desired by the surgeon to leave the dilation tube in place. It should also be noted that while threads are illustrated, other types of attachments may be utilized without departing from the scope of the invention; such attachments may include, but should not be limited to, adhesives, friction, thumbscrews, locking tapers, and the like. The inner bore of the dilation tube 166 is sized to cooperate with the tap tube 168 and may be sized for passage of an implant such as a pedicle screw or the like. The second end of the dilation tube is preferably tapered or radiused to allow traversal through tissue *in vivo.* The tap tube 168 and the dilation tube 166 are preferably constructed from a polymeric plastic material. However, it should be noted that metals, rubbers and suitable combinations thereof may be utilized without departing from the scope of the invention.

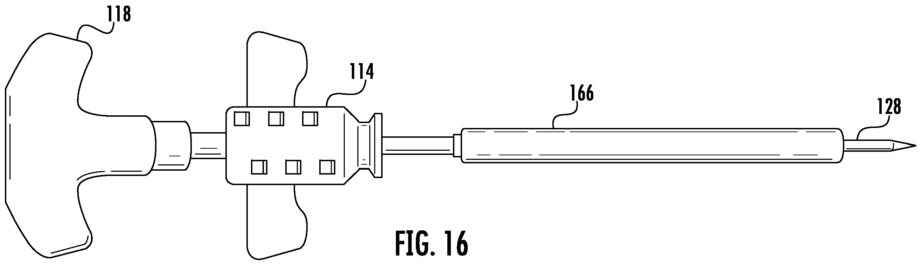

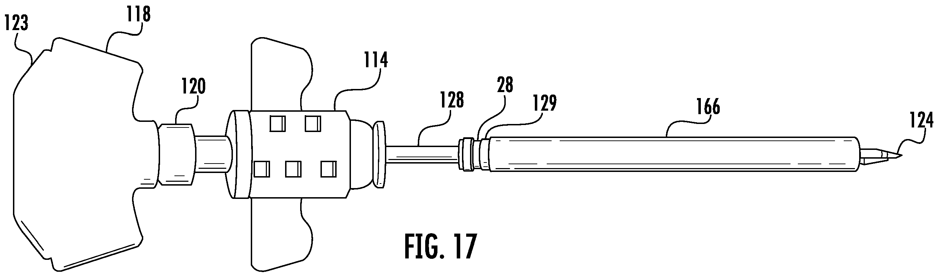

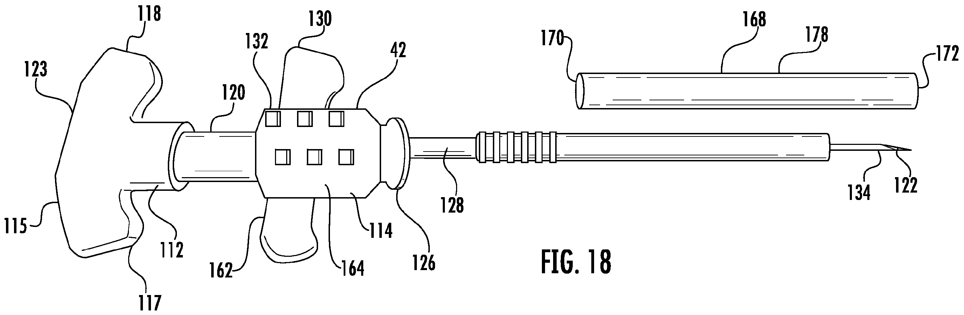

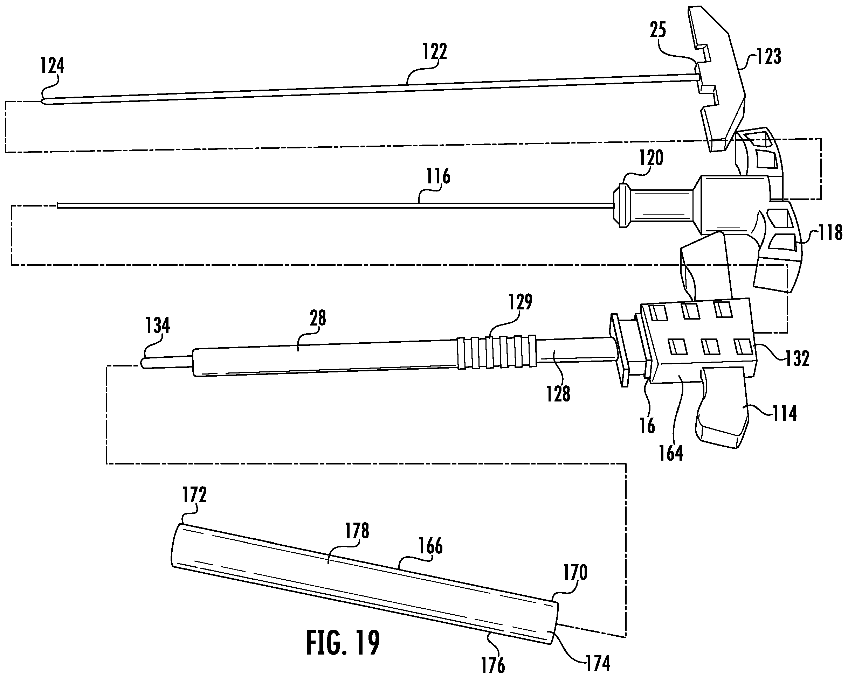

Figures 16-19 illustrate an alternate embodiment of the present invention. In these figures, a Jamshidi-type assembly 112 is slidably disposed within an outer cannula assembly 114. The Janshidi-type needle assembly 112 includes a cannula 116 which is secured to a handle 118. The handle 118 includes a threaded collar portion 120. This threaded collar portion 120 is fixedly secured or molded to the handle 118. The handle 118 preferably has an ergonomic shape that can comfortably fit into a surgeon's or medical technician's hand. The handle includes an upper curved portion 115 which is shaped to conform to an individual's palm. The lower portion 117 of the handle 118 is also curved. The curve of the lower portion 117 of the handle is designed to be grasped by the fingers of an individual to assist in the control of the Jamshidi-type assembly 112. The handle 118 is used to drive the cannula into, and sometimes through bones of a vertebra. Sometimes the Jamshidi-type assembly 112 can be driven through the bone only by using pressure exerted by an individual's hand. Other times a hammer or other instrument must be employed to drive the Jamshidi-type assembly 112 through a bone. There is a risk, that when a hammer or similar instrument is utilized, the Jamshidi-type assembly 112 will pass too far into a vertebra. This can cause damage to nerves located nearby. Sometimes the needle passes completely through the vertebra and injures an adjacent blood vessel or internal organ. To prevent this, the present invention utilizes a second cannula 114 which is adjustably secured to the Jamshidi-type assembly 112 so that the Jamshidi assembly can pass through the second cannula assembly 114 in a precisely controlled manner once the second cannula assembly is embedded into the desired bone for anchoring.

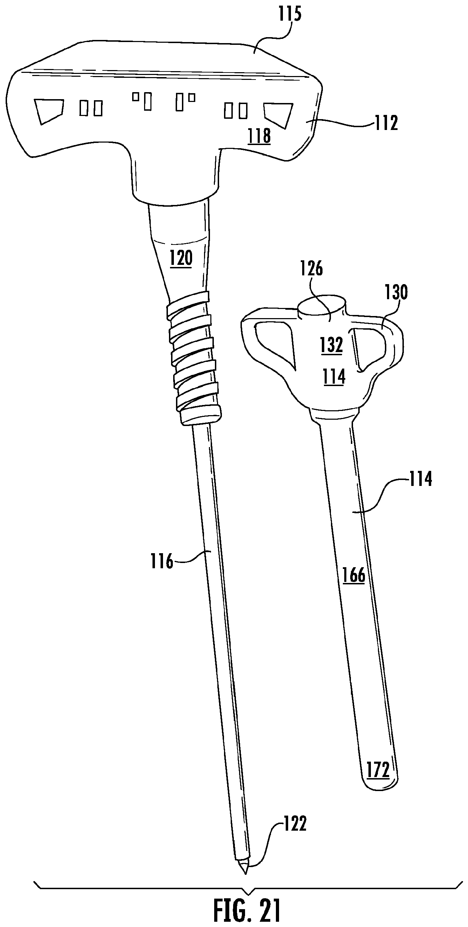

A needle 122 is slidably positioned within the cannula 116. The needle, like the other embodiments includes an enlarged head member 123 which includes a boss 25 (Fig. 21) threads or the like suitable to secure the needle 122 within the cannula 116. In a most preferred embodiment, the boss 25 cooperates with the handle 118 to function as a bayonet type mount. This embodiment of the present invention illustrates the needle as having a conical tip 124, however, other tips and needles can also be employed. For example, a trocar needle or point formed from a plurality of flat surfaces etc. can be utilized. The tip 124 can be solid, tapered, hollow, etc. The tip can be utilized to extract a tissue sample. It can also be utilized to anchor the needle to a bone. A drill, tap and/or orthopedic bone screw or other device (not shown) can then be passed down the needle and secured to the correct location on a bone. While the preferred embodiment of the present invention discloses a relatively rigid needle, other needles which are flexible can also be employed. Alternatively, the bayonet mount may be utilized to remove the needle from the inner cannula for placement of a guidewire, K-wire or a Kirschner wire.

The outer cannula 114 comprises an upper portion 126 secured to a cannula 128 (Fig. 8). The upper portion 126 comprises a handle or grip 130. In this embodiment, the outer or second cannula 114 and the handle 130 are fixedly secured to each other. In other embodiments, these elements can be pivotably secured to each other. The lower end 134 of the outer cannula 114 is constructed and arranged to penetrate and pass through bone. While the end 134 is normally a hollow tube with an end that tapers to a sharp edge, other edges can also be employed. For example, the edge can be serrated, saw toothed or sinusoidal. The smooth edge is preferably utilized when the needle assembly is driven straight into or through a bone. The serrations or waves are employed when additional effort is required to penetrate a bone or tissue.

The Jamshidi-type assembly 112 of this embodiment includes a threaded collar portion 120. The second cannula 114 includes a housing 132 at a top end thereof. The housing is relatively hollow and includes internal threads which match the external threads on sleeve portion 120. After the surgeon inserts the needle into a patient and through the cortical bone 36 of a vertebra (Fig. 7), the cannula 116 is inserted further into the vertebra by rotating the handle 118 and sleeve portion 120. Rotation of sleeve 120 moves the cannula 116 and needle further into the vertebra. The distance that these elements are moved can be measured along the sleeve portion 120 which is provided with windows 162 and/or indicia 164. The distance the needle needs to travel into the vertebra, which has been determined by radiography, will be measured along sleeve portion 120. The surgeon or medical technician can now rotate handle 118 and sleeve 120 until it reaches the desired mark of indicia on sleeve 120. At this point, the cannula 116 and Jamshidi-type assembly 112 are now exactly where they need to be positioned.

Still referring to Figures 16-19, the preferred embodiment of the present invention incudes at least one tap tube 168 positioned about the outer cannula 114. In a most preferred embodiment, the tap tube 168 is positioned about the stop member 28 of the outer cannula 114. The stop member 28 is preferably molded around the outer cannula 128 however, press fits, shrink fits, adhesives, fasteners and the like may also be utilized to secure the stop member in place without departing from the scope of the invention. In a most preferred embodiment, threads 129 are formed into the stop member for threaded cooperation with the tap tube 166. The tap tube includes a first end 170, a second end 172, an inner diameter 174 and an outer diameter 176. The tap tube generally includes a length that is slightly shorter than the outer cannula 128. In this manner, the tap tube or the stop member can function as a stop member for the outer cannula, whereby the progression of the outer cannula through bone is stopped when the second end 172 of the tap tube or distal end of the stop member contacts the outer surface of the bone. The inner diameter 174 of the tap tube 168 includes threads sized to cooperate with the threads on the stop member and is sized to allow passage of a drill or tap member (not shown). The second end 172 of the tap tube 168 is preferably tapered or radiused to allow for traversal through tissue *in vivo.* Thus, in operation, a surgeon can tap the depth controlled jamshidi needle 10 into a position wherein the second end 172 of the tap tube 168 contacts the bone. The surgeon can then rotate the handle 118 until the inner cannula 116 reaches the desired depth. The cap 123 of the needle 22 can be rotated to release the needle from the inner cannula 116. The needle is then withdrawn and a guidewire can be passed through funnel 17 and the inner cannula to the surgical site. The tap tube 168 can then be rotated to release it from the outer cannula 114 and for backing the outer cannula out of the bone while leaving the tap tube in position. Gripping surface 178 provides the surgeon with sufficient friction to allow the tap tube to be released from the outer cannula. While the gripping surface 178 is illustrated as a knurled surface, other grippable surfaces may be utilized without departing from the scope of the invention; such gripping surfaces may include, but should not be limited to, wrench flats, an enlarged diameter, polygons and the like. A dilation tube 166 may be utilized in some embodiments over the outer diameter of the tap tube 168. The dilation tube includes a first end 180, a second end 182, an inner diameter 184 and an outer diameter 186. The first end 180 includes an attachment, illustrated herein as external threads 188 (Fig. 15) for attachment to the outer cannula. This construction allows the dilation tube 166 to be released as desired by the surgeon to leave the dilation tube in place. It should also be noted that while threads are illustrated, other types of attachments may be utilized without departing from the scope of the invention; such attachments may include, but should not be limited to, adhesives, friction, thumbscrews, locking tapers, and the like. The inner bore of the dilation tube 166 is sized to cooperate with the tap tube 168 and may be sized for passage of an implant such as a pedicle screw or the like. The second end of the dilation tube is preferably tapered or radiused to allow traversal through tissue *in vivo.* The tap tube 168 and the dilation tube 166 are preferably constructed from a polymeric plastic material. However, it should be noted that metals, rubbers and suitable combinations thereof may be utilized without departing from the scope of the invention.

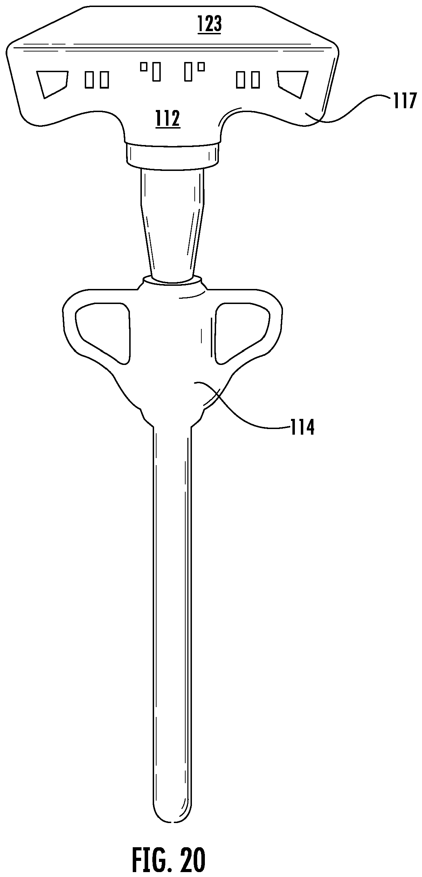

Referring to Figures 20-21, an alternative embodiment of the present invention is illustrated. In these figures, a Jamshidi-type assembly 112 is slidably disposed within an outer cannula assembly 114. The Jamshidi-type needle assembly 112 includes a cannula 116 which is secured to a handle 118. The handle 118 includes a threaded collar portion 120. This threaded collar portion 120 is fixedly secured or molded to the handle 118. The handle 118 preferably has an ergonomic shape that can comfortably fit into a surgeon's or medical technician's hand. The handle includes an upper curved portion 115 which is shaped to conform to an individual's palm. The lower portion 117 of the handle 118 is also curved. The curve of the lower portion 117 of the handle is designed to be grasped by the fingers of an individual to assist in the control of the Jamshidi-type assembly 112. The handle 118 is used to drive the cannula into, and sometimes through bones of a vertebra. Sometimes the Jamshidi-type assembly 112 can be driven through the bone only by using pressure exerted by an individual's hand. Other times a hammer or other instrument must be employed to drive the Jamshidi-type assembly 112 through a bone. There is a risk, that when a hammer or similar instrument is utilized, the Jamshidi-type assembly 112 will pass too far into a vertebra. This can cause damage to nerves located nearby. Sometimes the needle passes completely through the vertebra and injures an adjacent blood vessel or internal organ. To prevent this, the present invention utilizes a second cannula 114 which is adjustably secured to the Jamshidi-type assembly 112 via the threaded collar portion 120 so that the Jamshidi assembly can pass through the second cannula assembly 114 in a precisely controlled manner once the second cannula assembly is abutted to the desired bone for anchoring. Alternatively, the cannula 116 may be extended past a tap tube portion 166 of the second cannula 114 before be driven into the bone to limit the distance that the cannula 116 can penetrate the bone. Once the distal end 175 of the tap tube contacts the bone traversal of the cannula through the bone is stopped. Rotation of the jamshidi assembly in a first direction will cause further progression of the needle 122 through the bone or tissue while rotation in a second direction will pull the jamshidi assembly from the bone leaving the tap tube in position over the aperture created in the bone by the needle 122 and cannula 116.

A needle 122 is slidably positioned within the cannula 116. The needle, like the other embodiments includes an enlarged head member 123 which includes a boss 25 (Fig. 21) threads or the like suitable to secure the needle 122 within the cannula 116. In a most preferred embodiment, the boss 25 cooperates with the handle 118 to function as a bayonet type mount. This embodiment of the present invention illustrates the needle as having a conical tip 124, however, other tips and needles can also be employed. For example, a trocar needle or point formed from a plurality of flat surfaces etc. can be utilized. The tip 124 can be solid, tapered, hollow, etc. The tip can be utilized to extract a tissue sample. It can also be utilized to anchor the needle to a bone. A drill, tap and/or orthopedic bone screw or other device (not shown) can then be passed down the needle and secured to the correct location on a bone. While the preferred embodiment of the present invention discloses a relatively rigid needle, other needles which are flexible can also be employed. Alternatively, the bayonet mount may be utilized to remove the needle from the inner cannula for placement of a guidewire, K-wire or a Kirschner wire.

The outer cannula 114 comprises an upper portion 126 secured to a cannula 128 (Fig. 20). The upper portion 126 comprises a handle or grip 130. In this embodiment, the outer or second cannula 114 and the handle 130 are fixedly secured to each other. In other embodiments, these elements can be pivotably secured to each other. The lower end 134 of the outer cannula 114 is constructed and arranged to function as a tap tube 166 having an inner bore sized for the traversal of a tap member or drill bit (not shown) for sizing the aperture in the bone and/or forming threads therein for cooperation with a bone fastener (not shown).

The Jamshidi-type assembly 112 of this embodiment includes a threaded collar portion 120. The second cannula 114 includes a housing 132 at a top end thereof. The housing is relatively hollow and includes internal threads which match the external threads on sleeve portion 120. After the surgeon inserts the needle into a patient and through the cortical bone 36 of a vertebra (Fig. 7), the cannula 116 is inserted further into the vertebra by rotating the handle 118 and sleeve portion 120. Rotation of sleeve 120 moves the cannula 116 and needle further into the vertebra. The distance that these elements are moved can be measured along the sleeve portion 120 which may be provided with windows and/or indicia.

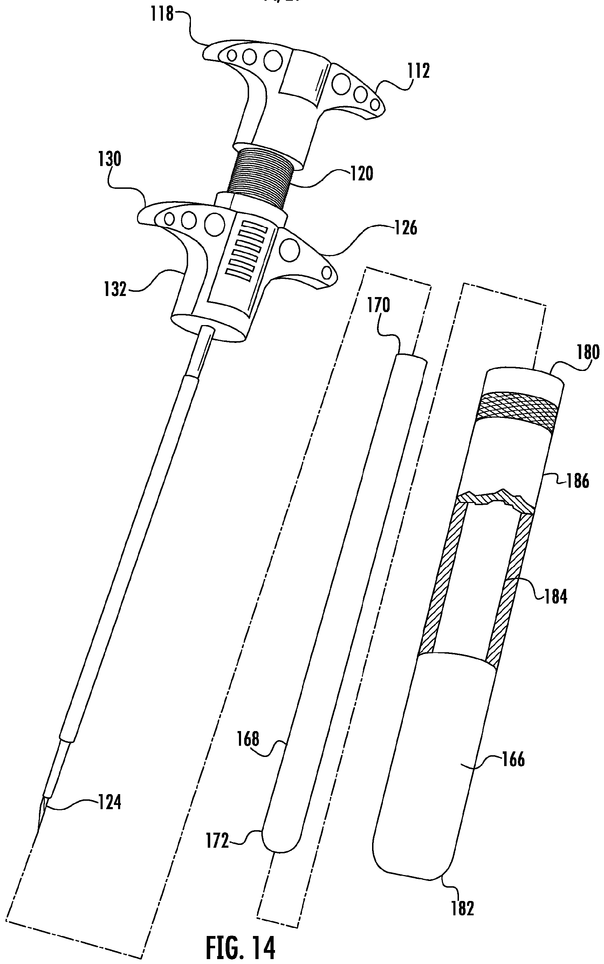

Still referring to Figures 20-21, the preferred embodiment of the present invention incudes at least one tap tube 168 positioned about the outer cannula 114. In a most preferred embodiment, the tap tube 168 is positioned to function as a stop member to prevent over insertion of the jamshidi assembly. In this manner, the tap tube can function as a stop member for the outer cannula, whereby the progression of the outer cannula through bone is stopped when the second end 172 of the tap tube or distal end of the stop member contacts the outer surface of the bone. The inner diameter 174 of the tap tube 168 is sized to allow passage of a drill or tap member (not shown). The second end 172 of the tap tube 168 is preferably tapered or radiused to allow for traversal through tissue *in vivo.* Thus, in operation, a surgeon can tap the depth controlled jamshidi needle 10 into a position wherein the second end 172 of the tap tube 168 contacts the bone. The surgeon can then rotate the handle 118 until the inner cannula 116 reaches the desired depth. The cap 123 of the needle 22 can be rotated to release the needle from the inner cannula 116. The needle is then withdrawn and a guidewire can be passed through funnel 17 and the inner cannula to the surgical site. The tap tube 168 can then be rotated to release it from the outer cannula 114 and for backing the inner cannula out of the bone while leaving the tap tube in position. Gripping surface 130 provides the surgeon with sufficient friction to allow the tap tube to be released from the inner cannula. While the gripping surface 178 is illustrated as a handle 130, other grippable surfaces may be utilized without departing from the scope of the invention; such gripping surfaces may include, but should not be limited to, wrench flats, an enlarged diameter, polygons and the like. A dilation tube 166 (Fig. 14) may be utilized in some embodiments over the outer diameter of the tap tube 168. The dilation tube includes a first end 180, a second end 182, an inner diameter 184 and an outer diameter 186. The first end 180 includes an attachment, illustrated herein as external threads 188 (Fig. 15) for attachment to the outer cannula. This construction allows the dilation tube 166 to be released as desired by the surgeon to leave the dilation tube in place. It should also be noted that while threads are illustrated, other types of attachments may be utilized without departing from the scope of the invention; such attachments may include, but should not be limited to, adhesives, friction, thumbscrews, locking tapers, and the like. The inner bore of the dilation tube 166 is sized to cooperate with the tap tube 168 and may be sized for passage of an implant such as a pedicle screw or the like. The second end of the dilation tube is preferably tapered or radiused to allow traversal through tissue *in vivo.* The tap tube 168 and the dilation tube 166 are preferably constructed from a polymeric plastic material. However, it should be noted that metals, rubbers and suitable combinations thereof may be utilized without departing from the scope of the invention.

All patents and publications mentioned in this specification are indicative of the levels of those skilled in the art to which the invention pertains.

It is to be understood that while a certain form of the invention is illustrated, it is not to be limited to the specific form or arrangement herein described and shown. It will be apparent to those skilled in the art that various changes may be made without departing from the scope of the invention and the invention is not to be considered limited to what is shown and described in the specification and any drawings/figures included herein.

One skilled in the art will readily appreciate that the present invention is well adapted to carry out the objectives and obtain the ends and advantages mentioned, as well as those inherent therein. The embodiments, methods, procedures and techniques described herein are presently representative of the preferred embodiments, are intended to be exemplary and are not intended as limitations on the scope. Changes therein and other uses will occur to those skilled in the art which are encompassed within the scope of the invention and are defined by the scope of the appended claims. Although the invention has been described in connection with specific preferred embodiments, it should be understood that the invention as claimed should not be unduly limited to such specific embodiments.

## Claims

1. A jamshidi assembly (10) comprising:
an outer cannula member (14) having the first portion of a helical thread type depth controlling assembly (32) secured thereto for adjustable cooperation with an inner cannula member,
an inner cannula member (16), said inner cannula member having a second portion of a helical thread type depth controlling assembly (42) secured thereto,
a needle member (22) removably secured within said inner cannula member (16) so that a tip portion (24) of said needle member extends beyond a distal end of said inner cannula member (16),
said outer cannula (14) having a second end (34) constructed and arranged to cooperate with a bone member to provide a stop member (28) against said bone, said first and said second portions of said depth controlling assembly threadably cooperating to provide user controlled depth of penetration of said needle member and said inner cannula member into said bone.

2. The jamshidi assembly of claim 1 wherein said stop member (28) is a tap tube (168) having an inner diameter (174) sized to pass a helical thread tap member when said outer cannula, said inner cannula and said needle member are removed prior to insertion of said tap member leaving said tap tube (168) in place at the surgical site.

3. The jamshidi assembly of claim 2 wherein said jamshidi assembly further includes a dilation tube (166), said dilation tube having an inner diameter (184) sized for positioning said dilation tube (166) around an outer diameter (176) of said tap tube (168).

4. The jamshidi assembly of claim 1 wherein said inner cannula includes a handle member (18), said handle member including said helical thread, said helical thread adapted to cooperate with helical thread of said outer cannula (14) to define said depth controlling assembly, whereby rotation of said handle member provides linear translation of said inner cannula member with respect to said outer cannula member.

5. The jamshidi assembly of claim 4 wherein said outer cannula assembly includes a handle member (30), said handle member including a helically threaded sleeve (32), said helical thread adapted to cooperate with said helical thread of said inner cannula (16), said inner cannula including a threaded nut (42) which is rotatably secured to said inner cannula, whereby rotation of said threaded nut without rotation of said handle member (18) provides linear translation of said inner cannula member with respect to said outer cannula member.

6. The jamshidi assembly of claim 4 wherein said inner cannula handle member (18) includes a funnel (17) which extends from the upper portion (15) of said handle (18) to a first end (13) of said inner cannula (16), said funnel (17) including an open mouth (19) which tapers substantially to the diameter of the inner bore of the inner cannula (16) providing the user with an easily targeted aperture for insertion of the needle (22).

## Patentansprüche

1. Jamshidianordnung (10), die Folgendes umfasst:
ein äußeres Kanülenelement (14), an dem der erste Abschnitt einer Tiefensteueranordnung (32) mit Schraubengewinde befestigt ist, zur einstellbaren Zusammenwirkung mit einem inneren Kanülenelement,
ein inneres Kanülenelement (16), wobei am inneren Kanülenelement ein zweiter Abschnitt einer Tiefensteueranordnung (42) mit Schraubengewinde befestigt ist,
ein Nadelelement (22), das im inneren Kanülenelement (16) derart entfernbar befestigt ist, dass sich ein Spitzenabschnitt (24) des Nadelelements über ein distales Ende des inneren Kanülenelements (16) hinaus erstreckt,
wobei die äußere Kanüle (14) ein zweites Ende (34) aufweist, das konstruiert und angeordnet ist, um mit einem Knochenelement zusammenzuwirken, um ein Anschlagelement (28) gegen den Knochen bereitzustellen, wobei der erste und der zweite Abschnitt der Tiefensteueranordnung schraubbar zusammenwirken, um eine benutzergesteuerte Penetrationstiefe des Nadelelements und des inneren Kanülenelements in den Knochen bereitzustellen.

2. Jamshidianordnung nach Anspruch 1, wobei das Anschlagelement (28) ein Gewindebohrrohr (168) mit einem Innendurchmesser (174) ist, der dimensioniert ist, um ein Gewindebohrelement mit Schraubengewinde hindurchzuführen, wenn die äußere Kanüle, die innere Kanüle und das Nadelelement vor dem Einführen des Gewindebohrelements entfernt werden und das Gewindebohrrohr (168) an der Operationsstelle verbleibt.

3. Jamshidianordnung nach Anspruch 2, wobei die Jamshidianordnung ferner ein Dilationsrohr (166) beinhaltet, wobei das Dilationsrohr einen Innendurchmesser (184) aufweist, der zum Positionieren des Dilationsrohrs (166) um einen Außendurchmesser (176) des Gewindebohrrohrs (168) dimensioniert ist.

4. Jamshidianordnung nach Anspruch 1, wobei die innere Kanüle ein Griffelement (18) beinhaltet, wobei das Griffelement das Schraubengewinde beinhaltet, wobei das Schraubengewinde angepasst ist, mit dem Schraubengewinde der äußeren Kanüle (14) zusammenzuwirken, um die Tiefensteueranordnung zu definieren, wodurch eine Drehung des Griffelements eine lineare Umsetzung des inneren Kanülenelements mit Bezug auf das äußere Kanülenelement bereitstellt.

5. Jamshidianordnung nach Anspruch 4, wobei die äußere Kanülenanordnung ein Griffelement (30) beinhaltet, wobei das Griffelement eine Hülse (32) mit Schraubengewinde beinhaltet, wobei das Schraubengewinde angepasst ist, mit dem Schraubengewinde der inneren Kanüle (16) zusammenzuwirken, wobei die innere Kanüle eine Gewindemutter (42) beinhaltet, die drehbar an der inneren Kanüle befestigt ist, wodurch eine Drehung der Gewindemutter ohne Drehung des Griffelements (18) eine lineare Umsetzung des inneren Kanülenelements mit Bezug auf das äußere Kanülenelement bereitstellt.

6. Jamshidianordnung nach Anspruch 4, wobei das Griffelement (18) der inneren Kanüle einen Trichter (17) beinhaltet, der sich vom oberen Abschnitt (15) des Griffs (18) zu einem ersten Ende (13) der inneren Kanüle (16) erstreckt, wobei der Trichter (17) einen offenen Mund (19) beinhaltet, der sich im Wesentlichen zum Durchmesser der inneren Bohrung der inneren Kanüle (16) verjüngt, wodurch dem Benutzer eine leicht angezielte Öffnung zum Einführen der Nadel (22) bereitgestellt wird.

## Revendications

1. Ensemble Jamshidi (10) comprenant :
un élément de canule externe (14) ayant la première partie d'un ensemble de contrôle de profondeur de type à filetage hélicoïdal (32) fixée à ce dernier pour la coopération ajustable avec un élément de canule interne,
un élément de canule interne (16), ledit élément de canule interne ayant une seconde partie d'un ensemble de contrôle de profondeur de type à filetage hélicoïdal (42) fixée à ce dernier,
un élément d'aiguille (22) fixé de manière amovible dans ledit élément de canule interne (16) de sorte qu'une partie de pointe (24) dudit élément d'aiguille s'étend au-delà d'une extrémité distale dudit élément de canule interne (16),
ladite canule externe (14) ayant une seconde extrémité (34) construite et agencée pour coopérer avec un élément d'os pour fournir un élément de butée (28) contre ledit os, lesdites première et seconde parties dudit ensemble de contrôle de profondeur coopérant par filetage pour fournir à l'utilisateur une profondeur de pénétration contrôlée dudit élément d'aiguille et dudit élément de canule interne dans ledit os.

2. Ensemble Jamshidi selon la revendication 1, dans lequel ledit élément de butée (28) est un tube taraudé (168) ayant un diamètre interne (174) dimensionné pour faire passer un élément de taraud à filetage hélicoïdal lorsque ladite canule externe, ladite canule interne et ledit élément d'aiguille sont retirés avant l'insertion dudit élément de taraud, laissant ledit tube taraudé (168) en place sur le site chirurgical.

3. Ensemble Jamshidi selon la revendication 2, dans lequel ledit ensemble Jamshidi comprend en outre un tube de dilatation (166), ledit tube de dilatation ayant un diamètre interne (184) dimensionné pour positionner ledit tube de dilatation (166) autour d'un diamètre externe (176) dudit tube taraudé (168).

4. Ensemble Jamshidi selon la revendication 1, dans lequel ladite canule interne comprend un élément de poignée (18), ledit élément de poignée comprenant ledit filetage hélicoïdal, ledit filetage hélicoïdal étant adapté pour coopérer avec le filetage hélicoïdal de ladite canule externe (14) afin de définir ledit ensemble de contrôle de profondeur, moyennant quoi la rotation dudit élément de poignée fournit la translation linéaire dudit élément de canule interne par rapport audit élément de canule externe.

5. Ensemble Jamshidi selon la revendication 4, dans lequel ledit ensemble de canule externe comprend un élément de poignée (30), ledit élément de poignée comprenant un manchon fileté de manière hélicoïdale (32), ledit filetage hélicoïdal étant adapté pour coopérer avec ledit filetage hélicoïdal de ladite canule interne (16), ladite canule interne comprenant un écrou fileté (42) qui est fixé, en rotation, sur ladite canule interne, moyennant quoi la rotation dudit écrou fileté sans rotation dudit élément de poignée (18) fournit la translation linéaire dudit élément de canule interne par rapport audit élément de canule externe.

6. Ensemble Jamshidi selon la revendication 4, dans lequel ledit élément de poignée de canule interne (18) comprend un entonnoir (17) qui s'étend à partir de la partie supérieure (15) de ladite poignée (18) jusqu'à une première extrémité (13) de ladite canule interne (16), ledit entonnoir (17) comprenant une bouche ouverte (19) qui se rétrécit progressivement jusqu'au diamètre de l'alésage interne de la canule interne (16) fournissant à l'utilisateur une ouverture facilement ciblée pour l'insertion de l'aiguille (22).
